# FASCICULE DE BREVET EUROPEEN

(11) **EP 2 147 982 B1**
(45) Date de publication et mention de la délivrance du brevet: **08.03.2017**
(21) Numéro de dépôt: 09166054.8
(22) Date de dépôt: 21.07.2009
(51) Int. Cl.: C12Q 1/68, G01N 33/53

(54) **Signature moléculaire représentative de dysfonctionnements de l'homéostasie épidermique**
Molekulare Signatur repräsentativ für eine dysfunktionelle epidermale Homöostasis
Molecular signature representing a dysfunctional epidermal homeostasis

(30) Priorité: 22.07.2008 FR 0804169; 09.09.2008 US 95360 P
(43) Date de publication de la demande: 27.01.2010
(73) Titulaire: L'Oréal, 75008 Paris (FR)
(72) Inventeur: Sextius, Peggy, 94120 Fontenay sous Bois (FR); Bernard, Bruno, 92400 Courbevoie (FR); Bernerd, Françoise, 75018 Paris (FR); Marionnet, Claire, 92410 Ville-D'Avray (FR)
(74) Mandataire: Sellin, Carole

(56) Documents cités:
- WO-A-2005/100603
- YE J ET AL: "Alterations in cytokine regulation in aged epidermis: implications for permeability barrier homeostasis and inflammation. I. IL-1 gene family." EXPERIMENTAL DERMATOLOGY JUN 2002, vol. 11, no. 3, juin 2002 (2002-06), pages 209-216, XP002522056 ISSN: 0906-6705
- CURTO E V ET AL: "Biomarkers of human skin cells identified using DermArray DNA arrays and new bioinformatics methods" BIOCHEMICAL AND BIOPHYSICAL RESEARCH COMMUNICATIONS, ACADEMIC PRESS INC. ORLANDO, FL, US, vol. 291, no. 4, 8 mars 2002 (2002-03-08), pages 1052-1064, XP002265763 ISSN: 0006-291X
- MARIONNET CLAIRE ET AL: "Modulation of gene expression induced in human epidermis by environmental stress in vivo." THE JOURNAL OF INVESTIGATIVE DERMATOLOGY DEC 2003, vol. 121, no. 6, décembre 2003 (2003-12), pages 1447-1458, XP002522057 ISSN: 0022-202X
- CHOI EUNG-HO ET AL: "Stratum corneum acidification is impaired in moderately aged human and murine skin." THE JOURNAL OF INVESTIGATIVE DERMATOLOGY DEC 2007, vol. 127, no. 12, décembre 2007 (2007-12), pages 2847-2856, XP002522058 ISSN: 1523-1747
- BARLAND CHANTEL O ET AL: "Imiquimod-induced interleukin-1 alpha stimulation improves barrier homeostasis in aged murine epidermis." THE JOURNAL OF INVESTIGATIVE DERMATOLOGY FEB 2004, vol. 122, no. 2, février 2004 (2004-02), pages 330-336, XP002522059 ISSN: 0022-202X
- GHADIALLY R ET AL: "The aged epidermal permeability barrier. Structural, functional, and lipid biochemical abnormalities in humans and a senescent murine model." THE JOURNAL OF CLINICAL INVESTIGATION MAY 1995, vol. 95, no. 5, mai 1995 (1995-05), pages 2281-2290, XP002522060 ISSN: 0021-9738
- ELIAS PETER M ET AL: "The aged epidermal permeability barrier: basis for functional abnormalities." CLINICS IN GERIATRIC MEDICINE FEB 2002, vol. 18, no. 1, février 2002 (2002-02), pages 103-120 , vii, XP009114232 ISSN: 0749-0690

### SEQUENCE LISTING

<110> L'OREAL
<120> Signature moléculaire représentative de dysfonctionnements de l'homéostasie épidermique
<130> B07674A- CA/CS
<150> FR08/04169
   <151> 2008-07-22
<150> US61/095,360
   <151> 2008-09-09
<160> 50
<170> PatentIn version 3.3
<210> 1
   <211> 20
   <212> DNA
   <213> Artificial
<220>
   <223> Amorce/primer
<400> 1
   cctatctcag ggagggaagg 20
<210> 2
   <211> 20
   <212> DNA
   <213> Artificial
<220>
   <223> Amorce/primer
<400> 2
   tctccaagtc ccactctgct 20
<210> 3
   <211> 20
   <212> DNA
   <213> Artificial
<220>
   <223> Amorce/primer
<400> 3
   ttggcagttt aggacctgct 20
<210> 4
   <211> 20
   <212> DNA
   <213> Artificial
<220>
   <223> Amorce/primer
<400> 4
   gttcttcctg gcttttgctg 20
<210> 5
   <211> 27
   <212> DNA
   <213> Artificial
<220>
   <223> Amorce/primer
<400> 5
   cttctcatca atacctgttc cactgag 27
<210> 6
   <211> 27
   <212> DNA
   <213> Artificial
<220>
   <223> Amorce/primer
<400> 6
   atcaggacaa ctgacttgtc agatgag 27
<210> 7
   <211> 20
   <212> DNA
   <213> Artificial
<220>
   <223> Amorce/primer
<400> 7
   cacatcacaa ccctcattgc 20
<210> 8
   <211> 20
   <212> DNA
   <213> Artificial
<220>
   <223> Amorce/primer
<400> 8
   cgaagtccag ctccagtttc 20
<210> 9
   <211> 20
   <212> DNA
   <213> Artificial
<220>
   <223> Amorce/primer
<400> 9
   aaagcttgcg gtgaggtaga 20
<210> 10
   <211> 20
   <212> DNA
   <213> Artificial
<220>
   <223> Amorce/primer
<400> 10
   cttgtcaagg agcgacttcc 20
<210> 11
   <211> 20
   <212> DNA
   <213> Artificial
<220>
   <223> Amorce/primer
<400> 11
   ctccgtgaga gctggaaaac 20
<210> 12
   <211> 20
   <212> DNA
   <213> Artificial
<220>
   <223> Amorce/primer
<400> 12
   cgtttccgtt tatggctgtt 20
<210> 13
   <211> 20
   <212> DNA
   <213> Artificial
<220>
   <223> Amorce/primer
<400> 13
   ccccaaccag acctctctgt 20
<210> 14
   <211> 20
   <212> DNA
   <213> Artificial
<220>
   <223> Amorce/primer
<400> 14
   tctgatggct caaacacagc 20
<210> 15
   <211> 20
   <212> DNA
   <213> Artificial
<220>
   <223> Amorce/primer
<400> 15
   tgggcagata tgacctgaca 20
<210> 16
   <211> 20
   <212> DNA
   <213> Artificial
<220>
   <223> Amorce/primer
<400> 16
   ttacgaagtg cccctgtacc 20
<210> 17
   <211> 20
   <212> DNA
   <213> Artificial
<220>
   <223> Amorce/primer
<400> 17
   caccaagcgt gtgaagaaga 20
<210> 18
   <211> 20
   <212> DNA
   <213> Artificial
<220>
   <223> Amorce/primer
<400> 18
   cctcagcctt gctaaacagg 20
<210> 19
   <211> 22
   <212> DNA
   <213> Artificial
<220>
   <223> Amorce/primer
<400> 19
   tccaacagcg aactggtaca ga 22
<210> 20
   <211> 19
   <212> DNA
   <213> Artificial
<220>
   <223> Amorce/primer
<400> 20
   gcatgcagta gcggccttt 19
<210> 21
   <211> 20
   <212> DNA
   <213> Artificial
<220>
   <223> Amorce/primer
<400> 21
   tgcaaccaat ttggaaaaca 20
<210> 22
   <211> 20
   <212> DNA
   <213> Artificial
<220>
   <223> Amorce/primer
<400> 22
   ttcatgggtt cccagaagag 20
<210> 23
   <211> 20
   <212> DNA
   <213> Artificial
<220>
   <223> Amorce/primer
<400> 23
   gcagccagaa cagaagttcc 20
<210> 24
   <211> 20
   <212> DNA
   <213> Artificial
<220>
   <223> Amorce/primer
<400> 24
   atcaggggtg tctgcatagg 20
<210> 25
   <211> 20
   <212> DNA
   <213> Artificial
<220>
   <223> Amorce/primer
<400> 25
   ctcctggcgt ctaaatgagc 20
<210> 26
   <211> 20
   <212> DNA
   <213> Artificial
<220>
   <223> Amorce/primer
<400> 26
   ggagcaatcc tttcattcca 20
<210> 27
   <211> 23
   <212> DNA
   <213> Artificial
<220>
   <223> Amorce/primer
<400> 27
   atgggtgtga caccaacgcg gcc 23
<210> 28
   <211> 23
   <212> DNA
   <213> Artificial
<220>
   <223> Amorce/primer
<400> 28
   gtagatacac tgggcccgct ggg 23
<210> 29
   <211> 20
   <212> DNA
   <213> Artificial
<220>
   <223> Amorce/primer
<400> 29
   ggttccacca catcctgact 20
<210> 30
   <211> 20
   <212> DNA
   <213> Artificial
<220>
   <223> Amorce/primer
<400> 30
   tgagcatgcc actaatggag 20 0
<210> 31
   <211> 20
   <212> DNA
   <213> Artificial
<220>
   <223> Amorce/primer
<400> 31
   tggcactgat ggctttgtaa 20
<210> 32
   <211> 20
   <212> DNA
   <213> Artificial
<220>
   <223> Amorce/primer
<400> 32
   caggtgatct tcccgttgtt 20
<210> 33
   <211> 20
   <212> DNA
   <213> Artificial
<220>
   <223> Amorce/primer
<400> 33
   aaacgcctcc ataacaccag 20
<210> 34
   <211> 20
   <212> DNA
   <213> Artificial
<220>
   <223> Amorce/primer
<400> 34
   aaccagtcac agccaaaacc 20
<210> 35
   <211> 20
   <212> DNA
   <213> Artificial
<220>
   <223> Amorce/primer
<400> 35
   ggtccagaga ccacaccact 20
<210> 36
   <211> 20
   <212> DNA
   <213> Artificial
<220>
   <223> Amorce/primer
<400> 36
   cctctgggga aagaatcctc 20
<210> 37
   <211> 26
   <212> DNA
   <213> Artificial
<220>
   <223> Amorce/primer
<400> 37
   gggcaagttc cgtgggcatc atgttg 26
<210> 38
   <211> 31
   <212> DNA
   <213> Artificial
<220>
   <223> Amorce/primer
<400> 38
   ccagtaactc agctactctt tgtggctttc t 31
<210> 39
   <211> 26
   <212> DNA
   <213> Artificial
<220>
   <223> Amorce/primer
<400> 39
   gctcctcggc tttgacagag tgcaag 26
<210> 40
   <211> 30
   <212> DNA
   <213> Artificial
<220>
   <223> Amorce/primer
<400> 40
   gcatttgtgt ccaggtcctc catgatgtgt 30
<210> 41
   <211> 20
   <212> DNA
   <213> Artificial
<220>
   <223> Amorce/primer
<400> 41
   agctttgtgg gggagaaagt 20
<210> 42
   <211> 20
   <212> DNA
   <213> Artificial
<220>
   <223> Amorce/primer
<400> 42
   atccatggca ggaagtcaag 20
<210> 43
   <211> 20
   <212> DNA
   <213> Artificial
<220>
   <223> Amorce/primer
<400> 43
   ctgctgacga tgatgaagga 20
<210> 44
   <211> 20
   <212> DNA
   <213> Artificial
<220>
   <223> Amorce/primer
<400> 44
   ctcccagcaa ggacagaaac 20
<210> 45
   <211> 20
   <212> DNA
   <213> Artificial
<220>
   <223> Amorce/primer
<400> 45
   gagaactcac tggccgagac 20
<210> 46
   <211> 20
   <212> DNA
   <213> Artificial
<220>
   <223> Amorce/primer
<400> 46
   ctgaagaggc ttccctgatg 20
<210> 47
   <211> 20
   <212> DNA
   <213> Artificial
<220>
   <223> Amorce/primer
<400> 47
   cattctgtct cccccaaaaa 20
<210> 48
   <211> 20
   <212> DNA
   <213> Artificial
<220>
   <223> Amorce/primer
<400> 48
   atgggggtat aagggagctg 20
<210> 49
   <211> 20
   <212> DNA
   <213> Artificial
<220>
   <223> Amorce/primer
<400> 49
   tgcagctgga tgactacctg 20
<210> 50
   <211> 20
   <212> DNA
   <213> Artificial
<220>
   <223> Amorce/primer
<400> 50
   gaagctctcc caggacacag 20

## Description

La présente invention est dans le domaine cosmétique, et plus particulièrement dans le domaine de la peau.
L'invention repose sur l'identification, par les inventeurs de 25 gènes dont la cinétique de modulation ou le niveau de modulation au sein de l'épiderme après une agression, reflète l'homéostasie épidermique de la peau et se distingue entre une peau dite « âgée » et une peau dite « jeune ».

Le stratum corneum, ou couche cornée, est la couche superficielle de l'épiderme située à l'interface entre l'organisme et son environnement. Il est composé de cornéocytes, cellules anucléées résultant de la différenciation des kératinocytes épidermiques. Les cornéocytes sont riches en kératines et sont entourés d'une matrice lipidique imperméable. De par sa composition en protéines et en lipides, le stratum corneum joue un rôle essentiel de barrière cutanée. Il empêche l'intrusion d'agents microbiologiques et permet de préserver l'hydratation de la peau et donc du corps en général.
Toutes les atteintes physiques ou chimiques à l'intégrité du stratum corneum ont pour conséquence une augmentation de la perte en eau au travers de la peau. Ainsi l'application topique d'acétone ou de détergents provoquant une délipidation superficielle du stratum corneum, ou le tape stripping, augmentent de manière très importante la perte insensible en eau (Grubauer, G et al, 1989 ; Kuss, O. et al, 1998 ; Pinnagoda, J et al, 1990 ; Tanaka, M. et al, 1997 ; Zhai H. et al, 1998). De telles altérations perturbent en fait toute l'homéostasie épidermique et y induisent une réponse physiologique dans le but de restaurer l'intégrité de la fonction barrière du stratum corneum. Ainsi, les kératinocytes des couches superficielles de l'épiderme activent la sécrétion extracellulaire de corps lipidiques afin d'apporter rapidement à la peau une nouvelle membrane imperméable et ainsi de réduire de façon superficielle la perte en eau (Grubauer, G. et al 1987 ; Menon, G.K., 1985).
D'autre part, la prolifération des kératinocytes de la couche basale est activée pour remplacer les cellules du stratum corneum qui ont été endommagées (Proksch E., 1991 ; Wood, L.CL et al, 1992).
La mesure de « Perte Insensible en Eau » (PIE) est la méthode la plus fréquemment utilisée pour évaluer l'intégrité de la fonction barrière du stratum corneum (Piepkorn, M. et al, 1994). La PIE se mesure à l'aide d'un évaporimètre. Cet appareil possède un détecteur d'humidité et de température qui lui permet de mesurer le gradient d'évaporation de l'eau à la surface de la peau. Cependant les mesures de PIE sont soumises à de nombreux facteurs de variations qui en limitent la précision. La température ambiante, l'hygrométrie, la turbulence de l'air ou la pression avec laquelle est appliqué l'appareil induisent en effet des variations notables au niveau de ces mesures (Barel, A.O. et al 1995). L'état de stress du volontaire lors des mesures a également une influence non négligeable, un temps de repos suffisant avant lecture est d'ailleurs conseillé (Van, S. et al 1994). Même sur des régions aussi semblables que les avant-bras, il a été montré une nécessité indispensable de randomisation des mesures, la PIE étant significativement accrue sur l'avant-bras dominant (Treffel, P. et al 1994). Dans ce contexte, la mise au point d'une méthode d'évaluation simple, fiable, reproductible et précise de l'état de la fonction barrière épidermique et par conséquence de la dynamique de sa fonction homéostasique, serait particulièrement avantageuse.
Concernant le vieillissement de la peau et en dehors des conséquences bien connues de l'âge sur le relief de la peau, de nombreux inconforts sont rapportés par les personnes âgées. Ces inconforts trouvent leur origine dans une altération de la fonction barrière et de l'homéostasie épidermique de leur peau. Il est notamment fréquemment observé chez les personnes âgées une plus forte prévalence aux xéroses chroniques, caractérisées par une perte en eau accrue au travers de la peau. En fait, le stratum corneum des peaux âgées a une teneur en lipides intercellulaires décrue par rapport aux peaux jeunes, particulièrement pendant la période hivernale. Ce changement de composition du stratum corneum en perturbe ses propriétés physico-chimiques de barrière cutanée. Le stratum corneum devient notamment plus sensible avec l'âge aux agressions physiques ou chimiques telles que le tape stripping ou l'application d'acétone (Ghadially, R. et al, 1995) et sa perméabilité aux médicaments et en particulier aux composés hydrophiles est décrue. D'autre part, la sévérité des allergies de contact est exacerbée sur les peaux âgées à cause d'un turnover épidermique plus lent donc moins fréquent (Piaserico, S. et al, 2004). Enfin, la vitesse de récupération de la fonction barrière après altération du stratum corneum est ralentie avec l'âge laissant supposer un dysfonctionnement de la fonction homéostasique de l'épiderme (Denda, M., 2002 ; Ghadially, R. et al, 1995 ; Leveque, J.L., 2001). Cependant, malgré ces perturbations fonctionnelles dues au vieillissement de la peau, il a été montré que le stratum corneum des personnes âgées a une épaisseur équivalente à celle des peaux jeunes (Lock-Andersen, J. et al, 1997) et une PIE constitutive peu différente (Ghadially, R. et al, 1996). Ceci démontre que la PIE ne permet pas, dans le cas précis du vieillissement, une évaluation complète de la fonctionnalité de la barrière cutanée et de l'homéostasie épidermique de la peau d'un sujet.
En revanche, la plupart des études cliniques décrites à ce jour se basent sur des mesures de PIE afin de mettre en lumière l'effet de traitements sur la fonction barrière cutanée ou pour suivre le retour de l'épiderme à un état homéostasique normal après une agression. Lors d'une étude visant à déterminer l'action de divers traitements cosmétiques sur la fonction de barrière de l'épiderme humain, il a été réalisé simultanément une analyse transcriptomique de la réponse de l'épiderme et des mesures de PIE (Marionnet C. et al, 2004). Il a été ainsi identifié des marqueurs reproductibles et communs aux différentes agressions, ainsi que des marqueurs spécifiques à chacune des agressions ; cependant la PIE n'a pas permis d'apporter des éléments de comparaison des traitements cosmétiques effectués.
Dans ces conditions, il existe un besoin d'identification de marqueurs pertinents, reproductibles et significatifs qui reflètent l'homéostasie épidermique et qui permettent de mettre en évidence un éventuel déséquilibre de la fonction barrière dû à l'homéostasie épidermique de la peau, tel que le déséquilibre dû à l'âge.

Dans cette optique, les inventeurs ont réalisé une étude transcriptomique comparative de la cinétique de retour à l'homéostasie épidermique de l'épiderme humain après altération du stratum corneum par tape stripping, entre des individus sains jeunes et d'autres âgés.
L'expression de plusieurs centaines de gènes a varié au cours du temps après tape stripping. Mais de façon surprenante et inattendue, les inventeurs ont observé que 18 gènes particuliers montrent une cinétique de modulation significativement différente selon l'âge du patient. Les inventeurs ont également démontré que 7 autres gènes montrent systématiquement à tous les temps, un niveau de modulation significativement différent après tape stripping en fonction de l'âge.
Les 25 gènes identifiés par les inventeurs permettent donc de distinguer une peau jeune d'une peau âgée en réponse à une agression, et donc par là d'évaluer la capacité homéostasique de l'épiderme d'un individu. Il s'agit d'une signature représentative des différences d'expression génique existant entre la peau humaine jeune et la peau âgée suite à une altération de la fonction barrière par une agression.

### Définitions :

Dans le cadre de la présente demande, les termes qui suivent revêtent plus particulièrement la signification suivante :
**Tape stripping** ou "décollement de la couche cornée par un adhésif" : Il s'agit d'une méthode pour réaliser une agression physique de la peau consistant à éliminer les cornéocytes à l'aide d'un ruban adhésif, le cas échéant en reproduisant plusieurs fois le décollement de l'adhésif. Le tape stripping est dit 'contrôlé' lorsqu'il n'altère que le stratum corneum et éventuellement les couches superficielles de l'épiderme malpighiens, sans altérer les couches plus profondes de l'épiderme.
**Homéostasie épidermique :** Caractérise l'ensemble des fonctions de régulation de l'épiderme qui lui confèrent la capacité de maintenir un équilibre physiologique et un bon fonctionnement en dépit des contraintes extérieures. L'homéostasie de l'épiderme est entre autres assurée par sa fonction barrière qui permet notamment d'empêcher l'intrusion d'agents microbiologiques et de préserver l'hydratation de la peau en empêchant la perte en eau. Cette capacité peut être excellente ou bonne quand, en réponse à une agression, l'épiderme réagit immédiatement ou rapidement pour reconstituer l'équilibre de fonctionnement, elle peut être déficiente ou inexistante quand le retour à l'équilibre est tardif ou n'est pas atteint. Cette capacité homéostasique de la peau peut donc être pleinement assurée, cas de la peau jeune normale, ou défective, cas de la peau âgée ou de toutes autres peaux présentant un déséquilibre de fonctionnement.

La présente invention est donc relative à l'utilisation d'un nouveau procédé d'évaluation clinique de l'état de l'épiderme à travers les déficiences de sa fonction homéostasique, par le biais de l'étude du niveau d'expression de tout ou partie des 25 gènes identifiés par les inventeurs.
Selon un premier aspect, la présente demande est relative à une méthode d'évaluation de l'homéostasie épidermique de la peau d'un sujet, plus particulièrement d'un être humain.
Selon une première mise en oeuvre, une telle méthode comprend l'analyse différentielle de l'expression des gènes ESRRA, CBX3, TRIO, TCRB, PRRG2, ICP22BP, GPX3, GBA, BPGM, SMT3H2, LGALS2, DOC1 et GSN, choisis parmi les gènes KRT6B, KRT16 (aussi connu sous l'appellation K16), ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN, en réponse à une agression physique ou chimique du stratum corneum.
Les 25 gènes précédemment cités, à savoir KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN, sont dénommés les gènes de l'invention. Ces gènes ne sont pas de la famille des cytokines de type IL1.
La présente invention concerne donc l'évaluation clinique de l'homéostasie épidermique et la mise en évidence de ses dysfonctionnements grâce à l'analyse différentielle de plusieurs gènes de l'invention en réponse à une agression, de préférence au moins deux gènes.
Parmi ces 25 gènes identifiés par les inventeurs, certains ont déjà été décrits comme étant spécifiques de l'épiderme. Il est cependant à noter qu'il n'avait jamais été mis en évidence avant l'invention que leur cinétique de modulation ou leur niveau de modulation, en réponse à une agression physique ou chimique, variait en fonction de l'âge de l'individu et permettait donc une évaluation de l'homéostasie épidermique.
Par ailleurs, parmi les gènes identifiés par les inventeurs, certains n'avaient jamais été décrits comme impliqués d'une quelconque manière dans l'homéostasie épidermique. Il s'agit des gènes ESRRA, CBX3, TRIO, TCRB, PRRG2, ICP22BP, GPX3, GBA, BPGM, SMT3H2, LGALS2, DOC1 et GSN. Ces gènes sont tout particulièrement préférés dans le cadre de la présente invention.
Selon une autre mise en oeuvre, ladite méthode comprend une étape de réalisation d'une agression du stratum corneum sur une région de la peau d'un individu, puis la détermination
- de la cinétique de modulation de l'expression des gènes ESRRA, CBX3, TRIO, TCRB, PRRG2, ICP22BP, GPX3, GBA, BPGM, SMT3H2, LGALS2 et DOC1, et
- du niveau de modulation de l'expression du gène GSN,
au sein de l'épiderme correspondant à la région agressée et une étape de comparaison de la cinétique ou du niveau de modulation observé à la cinétique de référence ou au niveau de référence pour la modulation de l'expression desdits gènes.

Selon encore une autre mise en oeuvre, ladite méthode comprend la comparaison de la cinétique de modulation de l'expression des gènes ESRRA, CBX3, TRIO, TCRB, PRRG2, ICP22BP, GPX3, GBA, BPGM, SMT3H2, LGALS2 et DOC1 au sein de l'épiderme correspondant à une région de la peau après une agression du stratum corneum et la comparaison du niveau de modulation de l'expression du gène GSN au sein de l'épiderme correspondant à une région de la peau après une agression du stratum corneum, ladite comparaison se faisant par rapport à la cinétique de référence ou au niveau de référence pour la modulation de l'expression desdits gènes.

Ledit sujet ou individu est un être humain, de préférence âgé d'au moins 20 ans, et tout particulièrement d'au moins 40 ans, de préférence d'au moins 60 ans. Il peut s'agir d'un homme ou d'une femme. La peau ou l'épiderme de ce sujet ou individu dont il est fait référence peut correspondre à n'importe quelle région du corps ; de préférence il s'agit de la peau ou de l'épiderme situé sur un des membres, et tout particulièrement de la peau ou de l'épiderme des bras, de préférence des avant bras.

Par analyse différentielle de l'expression d'un gène donné, on entend l'analyse des différences entre l'expression (ou bien la cinétique d'expression ou le taux d'expression) d'un gène au sein d'une région de peau ayant subi une agression par rapport à une région de peau du même individu n'ayant pas subi d'agression.

De même, par cinétique de modulation ou niveau de modulation d'un gène donné, on entend la différence entre la cinétique d'expression ou le niveau d'expression du gène au sein d'une peau agressée et celui au sein d'une peau non agressée. La peau agressée et non agressée est celle du même individu.

De préférence, les zones de peau agressée et non agressée sont des zones de peau équivalentes, en termes d'emplacement et de fonction ; par exemple, il s'agit de zones de peau situées sur des membres gauche et droit, en des endroits équivalents, notamment intérieur de l'avant bras.

Selon un mode de réalisation préféré de la méthode de l'invention, la modulation de l'expression du ou des gènes choisis est normalisée par référence à l'expression d'au moins un gène choisi parmi S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS au sein de l'épiderme correspondant à la région agressée De préférence la normalisation est effectuée à l'aide d'au moins un gène parmi RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS.

Les inventeurs ont en effet montré que le niveau d'expression de ces gènes était non seulement constant dans le temps qui suit l'agression, mais également semblable quel que soit l'âge de l'individu ; par conséquent, le niveau d'expression de ces gènes est parfaitement indépendant de l'homéostasie épidermique. Le niveau d'expression de ces gènes est donc adapté pour effectuer la normalisation de la cinétique ou du niveau d'expression d'un gène choisi selon la présente invention. Les 19 gènes précédemment cités peuvent être qualifiés de « gènes de ménage ».

Dans la méthode selon la présente invention, l'agression du stratum corneum qui est réalisée au niveau de la peau est une agression physique ou chimique. Selon une mise en oeuvre préférée plus particulièrement illustrée dans la section expérimentale de la demande, l'agression est réalisée par « tape stripping ». Selon d'autres mises en oeuvre, l'agression peut être chimique et consister par exemple en l'application d'acétone, d'un agent délipidant (par exemple SLS, SDS, ...) ou d'un agent chimique abrasif acide ou basique. L'agression peut également être réalisée par une méthode mécanique telle que l'abrasion ou l'utilisation de micro-aiguilles ; elle peut également être réalisée par une méthode physique telle que la chaleur.

D'autres moyens classiques pour effectuer une altération du stratum corneum sont bien connus de l'homme du métier dans le domaine de l'invention.

De préférence, l'agression du stratum corneum réalisée dans les méthodes de l'invention, entraîne une altération forte du stratum corneum. De manière tout particulièrement préférée, la très grande majorité du stratum corneum est endommagée, voire totalement retirée, sur la zone subissant l'agression.

Afin de limiter l'inconfort pour l'individu, la zone subissant l'agression peut toutefois être limitée à quelques centimètres carrés, voire moins. De préférence, la zone a un diamètre minimal de 3 mm.

De préférence, l'agression physique ou chimique du stratum corneum est limitée au stratum corneum et n'endommage pas les autres couches de l'épiderme, ni le derme. Dans la partie expérimentale de cette demande, il est décrit la réalisation d'un tape stripping contrôlé permettant de n'altérer que le stratum corneum. Les couches superficielles de l'épiderme malpighiens peuvent éventuellement être touchées, mais de préférence, l'agression est contrôlée pour éviter d'agir sur des couches plus internes de l'épiderme.

Pour les différents gènes de l'invention, dans certaines mises en oeuvre des méthodes de l'invention, il est fait mention de cinétique de référence ou de niveau de référence pour la modulation de l'expression du gène. Cette cinétique de référence ou niveau de référence correspond à la modulation de l'expression d'un gène de l'invention, qui est observée, après agression physique ou chimique de la peau, et caractérisant une homéostasie épidermique normale, c'est-à-dire une fonction barrière normale de l'épiderme, telle qu'on la trouve en général pour le peau de sujets jeunes.

De préférence, la cinétique de référence et/ou le niveau de référence pour la modulation des gènes de l'invention correspondent à ceux observés normalement pour la peau d'un sujet âgé d'environ 25 ans. Par « normalement », on entend la cinétique ou le niveau correspondant à la moyenne plus ou moins 10% des niveaux ou cinétiques d'au moins cinq, de préférence une dizaine, voire d'au moins un vingtaine voire une centaine d'individus âgés d'environ 25 ans.

Cette cinétique de référence et/ou ce niveau de référence pour la modulation d'un gène de l'invention peuvent être déterminés sur des volontaires jeunes, de préférence de moins de 25 ans, en fonction de l'agression réalisée. Les volontaires ont de préférence plus de 18 ans.

La partie expérimentale de la présente demande montre la détermination de la cinétique et du niveau de modulation des 25 gènes de l'invention chez des individus âgés de 25 ans (+/- 4 ans), la cinétique et le niveau de modulation observés peuvent constituer, au sens de la présente invention, une cinétique de référence et un niveau de référence, pour la modulation des gènes de l'invention en réponse à un tape stripping.

A l'inverse, il est possible de définir la cinétique de référence et/ou le niveau de référence pour la modulation des gènes de l'invention, par rapport à la modulation desdits gènes chez des individus présentant une peau dite âgée c'est-à-dire dont l'homéostasie épidermique, ou fonction barrière homéostasique, est altérée.

Par exemple, dans certaines mises en oeuvre de méthodes de l'invention, la cinétique de référence ou le niveau de référence correspond à la cinétique ou au niveau de modulation de l'expression dudit gène observé normalement pour une peau d'un sujet âgé d'environ 60 ans ou plus. Par normalement, on entend des cinétiques ou modulations observées « en moyenne », c'est-à-dire sur au moins 5, de préférence au moins 10, voire 20 ou 100 individus de plus de 60 ans.

Il est rappelé que dans le cadre de la présente invention, des gènes tout particulièrement préférés parmi les 25 gènes mis en évidence par les inventeurs, sont les gènes ESRRA, CBX3, TRIO, TCRB, PRRG2, ICP22BP, GPX3, GBA, BPGM, SMT3H2, LGALS2, DOC1 et GSN.

De ce fait, les méthodes de l'invention comprennent la comparaison de la cinétique de modulation de l'expression des gènes ESRRA, CBX3, TRIO, TCRB, PRRG2, ICP22BP, GPX3, GBA, BPGM, SMT3H2, LGALS2, DOC1 et la comparaison du niveau de modulation de l'expression du gène GSN.

Les présents inventeurs ont démontré qu'en réponse à une agression physique ou chimique du stratum corneum, la cinétique et/ou le niveau de modulation de ces 25 gènes différaient en fonction de la capacité homéostasique de l'épiderme de la peau examinée, ce qui constitue donc une signature des différences d'expression génique. Cependant, les modulations peuvent être différentes selon les individus ; de plus d'autres facteurs peuvent conduire à la modulation d'un gène de l'invention, sans pour autant que cette modulation soit nécessairement en rapport avec l'homéostasie épidermique.

Dans ces conditions, des méthodes sont mises en oeuvre en réalisant l'analyse différentielle ou la comparaison des niveaux ou cinétiques de modulation d'au moins deux gènes différents choisis parmi KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN. De préférence, au moins 3 gènes différents sont choisis et leur modulation suivie en réponse à une agression du stratum corneum. La combinaison d'au moins deux gènes, ou plus, peut tout particulièrement être sélectionnée parmi les gènes ESRRA, CBX3, TRIO, TCRB, PRRG2, ICP22BP, GPX3, GBA, BPGM, SMT3H2, LGALS2, DOC1 et GSN.

Selon d'autres mises en oeuvre préférées, les 25 gènes de l'invention sont choisis.

Des combinaisons sont notamment : au moins un gène parmi les gènes KRT6B et KRT16 combiné à au moins un gène parmi les gènes ESRRA, CBX3, MAP3K5, TRIO, PIK3CD, TCRB, PRRG2, ICP22BP, GPX3, GBA, BPGM, NID1, SMT3H2, LGALS2, DOC1, PRKCG et à au moins un gène parmi les gènes S100A8, S100A9, S100A2, S100A7, KRT15, SPRR1B et GSN.

Il est préféré qu'au moins deux gènes soient choisis. Selon une réalisation divulguée ici, au moins l'un des gènes de la combinaison est choisi parmi les gènes KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1 et PRKCG et au moins un gène de la combinaison est choisi parmi S100A8, S100A9, S100A2, S100A7, K15, SPRR1B et GSN. Selon une autre réalisation, au moins un gène est choisi parmi ESRRA, CBX3, TRIO, TCRB, PRRG2, ICP22BP, GPX3, GBA, BPGM, SMT3H2, LGALS2, DOC1 et au moins un autre gène est choisi parmi S100A8, S100A9, S100A2, S100A7, K15, SPRR1B et GSN.

Selon d'autres cas de figure, au moins trois gènes sont choisis parmi KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1 et PRKCG et au moins deux gènes sont choisis parmi S100A8, S100A9, S100A2, S100A7, K15, SPRR1B et GSN, ou bien au moins quatre gènes parmi la première liste et au moins trois gènes parmi la seconde liste.

De préférence, dans le cadre des méthodes selon l'invention l'analyse différentielle, la comparaison de la cinétique de modulation et/ou la comparaison des niveaux de modulation sont réalisés dans les 72 heures suivants l'agression chimique ou physique, notamment quand ladite agression est un tape stripping. En effet, c'est sur cet intervalle de temps que les inventeurs ont mis en évidence les différences les plus importantes entre les peaux jeunes et âgées et reflétant donc les dysfonctionnements de l'homéostasie épidermique. De préférence les analyses différentielles ou comparaisons mentionnées ci-dessus sont réalisées dans les 36 heures suivant l'agression chimique ou physique.

Comme explicité dans la partie expérimentale de la demande, les inventeurs ont de plus remarqué que les différences les plus significatives étaient observées 6 heures et 30 heures après l'agression chimique ou physique du stratum corneum.

Dans ces conditions, pour les gènes S100A8, S100A9, S100A2, S100A7, K15, SPRR1B et GSN, l'analyse différentielle ou la comparaison du niveau de modulation est de préférence réalisée 6 heures ou 30 heures après l'agression, ou bien successivement 6 et 30 heures après l'agression. Cette analyse ou comparaison peut sinon être réalisée à tout moment de préférence dans les 36 premières heures suivant l'agression du stratum corneum.

Pour les gènes choisis parmi KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1 et PRKCG, l'analyse différentielle ou la cinétique de modulation de l'expression du gène est déterminée de préférence 6 heures et 30 heures après l'agression physique ou chimique.

Les différentes méthodes selon la présente invention nécessitent la détermination de la modulation de l'expression des gènes ESRRA, CBX3, TRIO, TCRB, PRRG2, ICP22BP, GPX3, GBA, BPGM, SMT3H2, LGALS2, DOC1 et GSN, choisis parmi les 25 gènes de l'invention. L'expression des gènes est de préférence quantifiée par analyse de l'ARN messager (ARNm) transcrit au sein de l'épiderme. La quantification de l'ARNm transcrit est réalisée par exemple à l'aide de la technique de Northern Blot, dot blot, RT-PCR, hybridation sur puces à ADN, méthode SAGE ou par utilisation de cartes microfluidiques.

D'autres techniques permettant la quantification de l'ARNm peuvent bien entendu être utilisées. Toutes ces techniques sont bien connues de l'homme du métier.

Par exemple, des amorces pourront être utilisés pour l'analyse par RT-PCR de l'expression génique ayant les séquences suivantes illustrées dans le tableau A (séquences des amorces sens (S) et antisens (AS)).

**Tableau A :**

| | |
|---|---|
| ESRRA | S: cctatctcagggagggaagg (SEQ ID N°1), AS: tctccaagtcccactctgct (SEQ ID N°2) |
| CBX3 | S: ttggcagtttaggacctgct (SEQ ID N°3), AS: gttcttcctggcttttgctg(SEQ ID N°4) |
| KRT6B | S: cttctcatcaatacctgttccactgag (SEQ ID N°5), AS: atcaggacaactgacttgtcagatgag (SEQ ID N°6) |
| MAP3K5 | S: cacatcacaaccctcattgc (SEQ ID N°7), AS: cgaagtccagctccagtttc (SEQ ID N°8) |
| TRIO | S: aaagcttgcggtgaggtaga (SEQ ID N°9), AS: cttgtcaaggagcgacttcc (SEQ ID N°10) |
| PIK3CD | S: ctccgtgagagctggaaaac (SEQ ID N°11), AS: cgtttccgtttatggctgtt (SEQ ID N°12) |
| TCRB | S: ccccaaccagacctctctgt (SEQ ID N°13), AS: tctgatggctcaaacacagc(SEQ ID N°14) |
| PRRG2 | S: tgggcagatatgacctgaca (SEQ ID N°15), AS: ttacgaagtgcccctgtacc (SEQ ID N°16) |
| ICP22BP | S: caccaagcgtgtgaagaaga (SEQ ID N°17), AS: cctcagccttgctaaacagg (SEQ ID N°18) |
| K16 | S: tccaacagcgaactggtacaga (SEQ ID N°19), AS: gcatgcagtagcggccttt (SEQ ID N°20) |
| GPX3 | S: tgcaaccaatttggaaaaca (SEQ ID N°21), AS: ttcatgggttcccagaagag (SEQ ID N°22) |
| GBA | S: gcagccagaacagaagttcc (SEQ ID N°23), AS: atcaggggtgtctgcatagg (SEQ ID N°24) |
| BPGM | S: ctcctggcgtctaaatgagc (SEQ ID N°25), AS: ggagcaatcctttcattcca (SEQ ID N°26) |
| NID1 | S: atgggtgtgacaccaacgcggcc (SEQ ID N°27), AS: gtagatacactgggcccgctggg (SEQ ID N°28) |
| SMT3H2 | S: ggttccaccacatcctgact (SEQ ID N°29), AS: tgagcatgccactaatggag (SEQ ID N°30) |
| LGALS2 | S: tggcactgatggctttgtaa (SEQ ID N°31), AS: caggtgatcttcccgttgtt (SEQ ID N°32) |
| DOC1 | S: aaacgcctccataacaccag (SEQ ID N°33), AS: aaccagtcacagccaaaacc (SEQ ID N°34) |
| PRKCG | S: ggtccagagaccacaccact (SEQ ID N°35), AS: cctctggggaaagaatcctc (SEQ ID N°36) |
| S100A8 | S: gggcaagttccgtgggcatcatgttg (SEQ ID N°37), |
| | AS: ccagtaactcagctactctttgtggctttct (SEQ ID N°38) |
| S100A9 | S: gctcctcggctttgacagagtgcaag (SEQ ID N°39), |
| | AS: gcatttgtgtccaggtcctccatgatgtgt (SEQ ID N°40) |
| S100A2 | S: agctttgtgggggagaaagt (SEQ ID N°41), AS: atccatggcaggaagtcaag (SEQ ID N°42) |
| S100A7 | S: ctgctgacgatgatgaagga (SEQ ID N°43), AS: ctcccagcaaggacagaaac (SEQ ID N°44) |
| KRT15 | S: gagaactcactggccgagac (SEQ ID N°45), AS: ctgaagaggcttccctgatg (SEQ ID N°46) |
| SPRR1B | S: cattctgtctcccccaaaaa (SEQ ID N°47), AS: atgggggtataagggagctg (SEQ ID N°48) |
| GSN | S: tgcagctggatgactacctg (SEQ ID N°49), AS: gaagctctcccaggacacag (SEQ ID N°50) |

Il est également possible de quantifier le taux de transcription des gènes par l'analyse du taux de traduction et donc la quantification de protéines produites. Des techniques appropriées à cet objectif sont également bien connues de l'homme du métier. Par conséquent la signature moléculaire mise en évidence par les inventeurs se reflète également au niveau des protéines codées par les 25 gènes de l'invention.

L'analyse du taux de transcription des gènes de l'invention est réalisée au sein de l'épiderme, sous le stratum corneum ayant subi l'agression. A cet effet, des prélèvements d'épiderme ou de peau sont réalisés par exemple par la méthode décrite dans Marionnet C. et al, 2003, (prélèvements au dermatome, qui est un instrument permettant de prélever des bandes de peau très fines, de quelques dixièmes de millimètres d'épaisseur) ou toutes autres méthodes de prélèvement (biopsies, punch, kératome ou micro-kératome...).

Comme décrit dans ce qui précède, les 25 gènes de l'invention constituent donc une signature moléculaire de la peau, représentative des différences d'expression génique existant entre la peau humaine jeune et la peau âgée suite à une altération de la fonction barrière par une agression chimique ou physique, telle que par exemple tape stripping.

Cette signature moléculaire permet donc l'évaluation de la fonction homéostasique d'une peau humaine.

La présente invention a donc également pour objet l'utilisation d'une analyse de l'expression génique des gènes ESRRA, CBX3, TRIO, TCRB, PRRG2, ICP22BP, GPX3, GBA, BPGM, SMT3H2, LGALS2, DOC1 et GSN, dans un but de diagnostic de la fonction homéostasique de la peau, le niveau d'expression après tape stripping de tout ou partie de ces gènes après quelques heures, préférentiellement à 06h ou à 30h, étant caractéristique de l'âge de l'individu ou des déficiences de sa fonction homéostasique.

La technique consiste à suivre après quelques heures, de préférence à 6h et/ou 30h l'expression des gènes de la liste 1 consistant en ESRRA, CBX3, TRIO, TCRB, PRRG2, ICP22BP, GPX3, GBA, BPGM, SMT3H2, LGALS2 et DOC1 et du gène GSN, par toute technique de quantification de l'expression d'ARNm telles que le northern blot, dot blot, RT-PCR, hybridation sur puces à ADN, méthode SAGE, utilisation de cartes microfluidiques... L'étude transcriptomique de ces gènes est rendue possible grâce à une standardisation à l'aide des gènes de ménages identifiés et listés dans la liste 3 consistant en S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS.

Par exemple, des amorces pourront être utilisés pour l'analyse par RT-PCR de l'expression génique correspondant à celles illustrées dans le tableau A.

La présente invention concerne également des méthodes de diagnostic, permettant de mettre en évidence des déficiences au niveau de l'homéostasie épidermique d'une peau humaine, telles que les déficiences observées pour une peau âgée. Un tel diagnostic de l'état de l'épiderme d'un individu humain comprend l'évaluation de la fonction homéostasique ou homéostasie épidermique de la peau dudit individu selon l'une des méthodes décrites plus haut. Par cette méthode, la signature génique mise en évidence par les inventeurs permet de déceler des individus dont la fonction barrière et/ou l'homéostasie de la peau présentent des déficiences et qui de ce fait sont fragilisées. Un tel diagnostic peut donc permettre de conclure à la nécessité de traiter la peau pour en renforcer l'homéostasie et/ou la fonction barrière épidermique et ainsi les préserver. En effet, une déficience dans l'homéostasie épidermique est synonyme de fragilisation de la peau, et d'inconforts comme ceux dont souffrent les personnes âgées.

De préférence, le diagnostic est réalisé sur un individu âgé d'au moins 20, voire d'au moins 30, 40, 50, 55 ou 60 ans. La méthode de diagnostic selon la présente invention peut également être appropriée pour des personnes ayant subi des traitements importants susceptibles d'avoir modifié l'homéostasie épidermique, notamment des traitements topiques.

Selon cette méthode de diagnostic de l'invention, la peau diagnostiquée est qualifiée de « peau âgée » si l'analyse différentielle de l'expression d'au moins l'un , de préférence au moins deux des gènes choisis parmi ESRRA, CBX3, TRIO, TCRB, PRRG2, ICP22BP, GPX3, GBA, BPGM, SMT3H2, LGALS2, DOC1, et GSN s'écartent de plus de 50% de la modulation moyenne normalement observée pour une peau d'un sujet âgé d'environ 25 ans, de préférence 30 heures après l'agression physique ou chimique. La modulation moyenne normalement observée pour un individu d'environ 25 ans correspond à la modulation dite de référence.

La différence d'expression entre un gène de l'invention et la modulation moyenne est calculée de la façon suivante : [Intensité de modulation observée - Intensité de modulation de référence] / Intensité de modulation de référence. Comme expliqué dans les parties précédentes, l'intensité de modulation est normalisée à l'aide du niveau d'expression des gènes dits de ménages qui sont choisis parmi les gènes: S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS, de préférence choisis parmi RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS.

De préférence l'analyse différentielle de l'expression d'au moins l'un des gènes s'écarte de plus de 60% de la modulation de référence, voire de plus de 90% de la modulation de référence.

Selon des modes de réalisation tout particulièrement préférés, il est conclu à une peau ayant un comportement de « peau âgée » si au moins 8 ou 10, 12 ou 15 des gènes de l'invention présentent une modulation fortement différente de la modulation de référence, voire les 25 gènes.

Un autre but de la présente invention est relatif à l'utilisation d'une analyse de l'expression des gènes décrits dans la présente invention pour évaluer l'effet anti-âge d'un produit (actif, molécule, extrait naturel, combinaison d'actifs), mais aussi d'un procédé (lumière, injection, voie orale), seul ou combinés, en testant leur capacité à normaliser la réponse de l'épiderme à une agression. L'apport bénéfique d'un tel produit ou procédé anti-âge se caractériserait par sa capacité à
i) inhiber l'induction à 30h de tout ou partie des gènes ESRRA, CBX3, TRIO, TCRB, PRRG2, ICP22BP, GPX3, GBA, BPGM, SMT3H2, LGALS2 et, DOC1 après tape stripping chez les individus âgés, et/ou
ii) normaliser l'intensité de modulation du gène GSN chez les individus âgés.

La présente invention concerne donc également un procédé de détermination de l'efficacité d'un traitement de l'épiderme d'un individu, comprenant l'évaluation de la fonction homéostasique de la peau avant et après traitement par une méthode telle que décrite plus haut. En effet, la signature représentative des différences d'expression génique suite à une altération du stratum corneum peut être utilisée pour déterminer l'efficacité d'un traitement de l'épiderme. A cette fin, il faut déterminer ladite signature avant et après la mise en oeuvre du traitement dont on cherche à déterminer l'efficacité, de préférence sur une peau âgée.

De préférence, le procédé de détermination de l'efficacité d'un traitement est mis en oeuvre sur la peau d'un individu âgé d'au moins 20 ou 30 ans, de préférence d'au moins 40 ans, voire d'au moins 50 ou 60 ans.

Grâce à cette méthode, il est ainsi possible de déterminer la capacité de certains traitements, notamment des compositions cosmétiques pour application topique sur la peau ou des compléments alimentaires, ou de certains procédés de cosmétique instrumentale (lumière, injection, iontophorèse,...) à renforcer l'homéostasie épidermique et donc leur capacité à réaliser un « rajeunissement » de la peau. Par « rajeunissement » de la peau, on entend l'amélioration de l'homéostasie épidermique telle que le comportement de la peau en réponse à une agression redevient plus proche du comportement d'une peau jeune en réponse à cette même agression.

La présente invention concerne également un procédé de criblage d'actifs pour leur action bénéfique sur l'homéostasie épidermique de l'épiderme humain, comprenant l'évaluation de l'homéostasie épidermique de la peau d'un individu par une méthode selon telle que décrite précédemment, avant et après application de la molécule à cribler.

Comme détaillé plus haut, la signature représentative mise en évidence par les inventeurs permet en effet de mettre en évidence l'amélioration de l'homéostasie épidermique et/ou la fonction barrière des peaux âgées. Une telle amélioration se caractérise précisément par une cinétique de modulation ou un niveau de modulation après traitement plus proche de la cinétique de modulation ou le niveau de modulation observé pour la peau d'un sujet jeune.

La présente invention a également pour objet un procédé pour tester des traitements pour leur effet bénéfique sur l'homéostasie épidermique humaine, comprenant l'évaluation de l'homéostasie épidermique de la peau d'un individu par une méthode telle que décrite plus haut, avant et après mise en oeuvre du traitement à tester. L'utilisation de la signature moléculaire définie par les inventeurs à cette fin, est identique à celle détaillée plus haut pour le procédé de criblage de molécules pour leur action bénéfique sur l'homéostasie épidermique.

De préférence, un procédé de test tel que décrit est mis en oeuvre sur un individu âgé d'au moins 20 ou 30 ans, de préférence d'au moins 40 ans, et encore préférentiellement d'au moins 50 voire 60 ans.

Les traitements qui peuvent ainsi être évalués pour leur action positive sur l'homéostasie épidermique sont par exemple l'application locale d'ondes électromagnétiques ou de toutes composition, produit ou molécule chimique, ou bien l'injection ou l'ingestion d'une composition, produit ou molécule chimique. De préférence, il s'agit d'un traitement local topique.

Par produit, on entend aussi bien un ingrédient ou un actif sous une forme plus ou moins purifiée, notamment une molécule chimique, présentant une activité intrinsèque *in vitro* ou *in vivo*, qu'une formulation comprenant un ou plusieurs de ces ingrédients et un support et des adjuvants adaptés à l'application visée.

Dans les procédés d'évaluation de l'efficacité selon la présente invention, le traitement ou la molécule testée est considéré comme efficace s'il est observé :
- une inhibition de l'induction après 30 heures d'au moins un gène choisi parmi ESRRA, MAP3K5, TRIO, TCRB, PRRG2, ICP22BP, GPX3, GBA, BPGM, SMT3H2, LGALS2 et DOC1 ; et/ou
- une modification de l'intensité de modulation du gène GSN,

ladite modification réduisant la différence entre l'intensité de modulation observée et l'intensité de modulation de référence caractéristique de la peau d'un sujet âgé d'environ 25 ans.

De préférence également, l'inhibition de l'induction ou la modification de l'intensité de modulation observée après traitement représente une variation d'au moins 30% après application de la molécule ou du traitement. De préférence, les différences observées sont même supérieures, de préférence au moins 50%, voire plus, par exemple au moins 60%.

Du fait que les inventeurs ont déterminé une signature représentative de l'homéostasie épidermique, il est également possible, par les méthodes d'évaluation de la présente demande, d'objectiver l'action bénéfique d'un traitement, notamment d'un produit cosmétique, par exemple d'une composition cosmétique. Les méthodes d'évaluation décrites plus haut peuvent en effet être utilisées dans un protocole de test permettant de déterminer les produits susceptibles d'être qualifiés de « actifs sur la fonction barrière de la peau » ou bien « ayant un effet anti-âge » ou « effet de rajeunissement de la peau ».

Par ailleurs, au moyen de ce test, il est possible de promouvoir le produit auprès de consommateurs, en mettant en avant les résultats obtenus avec ce produit dans les méthodes d'évaluation de l'homéostasie épidermique décrites dans la présente invention.

L'évaluation de l'homéostasie épidermique sera basée sur l'étude de l'expression des gènes de l'invention ou des protéines encodées par ces gènes. La présente invention fournit donc également une méthode permettant de recommander un produit en signalant son effet dans un protocole de test constitué par une méthode d'évaluation telle que décrite précédemment. L'invention a donc également pour objet une méthode pour la promotion d'un produit cosmétique consistant à faire état d'une efficacité, action ou propriété dudit produit démontrée par au moins un test opéré tel que décrit précédemment.

Une telle promotion du produit pourra se faire par n'importe quel canal de communication.

Elle pourra être faite notamment par la vendeuse, directement sur le point de vente, par la radio et la télévision, notamment dans le cadre de spots publicitaires. Elle pourra être faite également par le canal de la presse écrite, ou par le biais de tout autre document, en particulier à des fins publicitaires (prospectus). Elle pourra se faire également par INTERNET, ou par tout autre réseau informatique adéquat. Elle pourra être faite également directement sur le produit, notamment sur son packaging ou sur toute notice explicative qui peut lui être associée.

La présente description décrit également un kit permettant de déterminer l'état plus ou moins performant de la fonction homéostatique d'une peau sur la base de l'analyse de l'expression des gènes constituant la signature représentative des différences d'expression génique existant entre la peau humaine jeune et la peau âgée, suite à une altération de la fonction barrière, par exemple par tape stripping. Ledit kit pour l'évaluation de l'homéostasie épidermique de la peau d'un sujet, comprend un moyen pour réaliser une agression physique ou chimique du stratum corneum, un moyen pour réaliser un prélèvement d'épiderme qui est de préférence distinct du premier, et un moyen pour déterminer le niveau d'expression d'au moins un gène choisi parmi les gènes KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1 B et GSN.

Le kit comprend au moins un moyen pour déterminer le niveau d'expression d'au moins un gène choisi parmi les gènes ESRRA, CBX3, TRIO, TCRB, PRRG2, ICP22BP, GPX3, GBA, BPGM, SMT3H2, LGALS2, DOC1 et GSN.

De préférence, pour les raisons mentionnées précédemment dans la demande, le kit comprend des moyens permettant de déterminer le niveau d'expression d'au moins 2, 3, 5, 10 ou au moins 15 gènes différents choisi parmi KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN, et tout particulièrement parmi ESRRA, CBX3, TRIO, TCRB, PRRG2, ICP22BP, GPX3, GBA, BPGM, SMT3H2, LGALS2, DOC1 et GSN. Il peut s'agir de moyens permettant de déterminer le niveau d'expression des 25 gènes de l'invention.

Le kit peut bien entendu comprendre des éléments supplémentaires, tels que différents contrôles, positifs ou négatifs. Avantageusement, un kit comprend également un moyen pour déterminer le niveau d'expression d'au moins un gène choisi parmi les gènes dits de ménage, c'est-à-dire parmi les gènes S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS.

De préférence, un kit permet de réaliser deux prélèvements épidermiques et permet de déterminer au moins deux fois le niveau d'expression du gène choisi, avant altération du corneum stratum et après, ou bien sur une zone agressée et sur une zone non-agressée.

Le kit peut également comprendre des instructions concernant son utilisation et les temps à respecter entre les différents prélèvements et l'altération du stratum corneum.

La présente description divulgue également une puce à ADN ou ARN comprenant des sondes s'hybridant spécifiquement avec l'ADNc ou ARNm des gènes KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1 B et GSN.

Une puce préférée dans le cadre de la présente description comprend des sondes s'hybridant spécifiquement avec l'ADNc ou l'ARNm des gènes ESRRA, CBX3, TRIO, TCRB, PRRG2, ICP22BP, GPX3, GBA, BPGM, SMT3H2, LGALS2, DOC1 et GSN.

Dans un aspect, une telle puce comprend également des sondes s'hybridant avec tout ou partie des ADNc ou ARNm des gènes S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS.

L'utilisation de ces puces dans le cadre des différentes méthodes de l'invention apparaît clairement à la lecture de ce qui précède pour l'homme du métier.

Dans toutes les mises en oeuvre de cette invention, le sujet est de préférence un être humain.

L'invention va maintenant être décrite plus en détails dans une de ces mises en oeuvre.

### Légende des figures :

**Figure 1** **:** Profil d'expression des 18 gènes dont la cinétique de modulation entre 6h et 30h après tape stripping est significativement différente selon l'âge du patient :
   **Figure 1A****)** Gènes dont l'expression est modulée à 06h et à 30 dans les deux populations mais dont l'allure cinétique est différente selon la population.
   **Figure 1B****)** Gènes dont l'expression croît entre 06h et 30h chez le sujet âgé alors qu'elle ne varie pas chez le sujet jeune.
**Figure 2** **:** Profil d'expression des 7 gènes dont l'intensité de modulation après tape stripping est significativement différente selon l'âge du patient quelque soit le temps.
**Figure 3** **:** Profil d'expression de 4 gènes dont l'intensité de modulation après tape stripping ne varie pas de façon significative selon l'âge du patient quelque soit le temps.

### EXEMPLES :

### 1. Méthodologie

Des volontaires masculins sains ont été recrutés afin de participer à une étude transcriptomique de plus de 4000 gènes connus pour être exprimés dans la peau.

Recrutement des volontaires : 5 groupes de 6 individus jeunes (25 +/- 4 ans) et 5 groupes de 6 individus âgés (67+/- 4 ans) ont été recrutés pour cette étude, après signature d'un consentement éclairé.

Altération du stratum corneum : Un tape stripping a été réalisé sur la face interne de l'un de leurs avant bras, choisi de façon randomisée, jusqu'à ce que le stratum corneum soit entièrement enlevé et que la peau présente un aspect lisse et brillant (48 +/- 7 strips en moyenne). Pour cela, un ruban adhésif 3M™ Blenderm™ a été utilisé (St Paul, MI, USA). Le second avant bras n'a subi aucun traitement.

Prélèvements d'épiderme: Des échantillons d'épiderme d'une superficie de 1.5 cm X1.5 ont été prélevés sous l'anesthésie locale à l'aide d'un dermatome GA630 (AESCULAP, Melsungen, Allemagne). Les prélèvements ont eu lieu sur peau strippées et non strippées. L'épaisseur de prélèvement a été fixée à 200 µm selon le protocole décrit par Marionnet et al en 2003, afin de ne prélever que l'épiderme et réduire au minimum la contamination dermique. Les prélèvements ont été effectués sur les deux avant-bras 02h, 06h, 19h, 30h et 72h après stripping.

Extraction d'ARNs: les échantillons d'épiderme ont été placés dans le tampon de lyse Rneasy (Qiagen, Courtaboeuf,France) immédiatement après prélèvement. Les tissus ont alors été broyés à l'aide d'un mortier stérile. Le lysat tissulaire a été homogénéisé (colonnes QIAshredder, Qiagen, Courtaboeuf, France) et les ARN totaux ont été extraits selon la méthode Rneasy (Qiagen, Courtaboeuf, la France). Une digestion de l'ADN a été effectuée pour éliminer toute contamination génomique (DNAse1, Qiagen, Courtaboeuf, la France). La concentration et la pureté des ARN totaux ont été déterminées par mesure d'absorbance à 260 nm et 280 nm. L'intégrité de l'ARN a été vérifiée par l'électrophorèse sur gel d'agarose après marquage au bromure d'Ethidium. Les échantillons d'ARN ont alors été précipités à l'aide d'une solution acide fortement saline (0.1 volume d'acétate de sodium 2M, pH4) et d'éthanol (2.5 volumes d'éthanol froid 100 %). Les échantillons ont été stockés à -80°c jusqu'à leur utilisation.

Hybridations sur Dermarray cDNA microarray : Des aliquotes correspondants à 2.5 µg d'ARN ont été prélevés et précipités par centrifugation. Les ARN ont été lavés, séchés puis solubilisés dans de l'eau stérile RNAse free. Des Oligos (dT) 12-18 mer, un mélange de dATP, dTTP et dGTP, l'AMV reverse transcriptase (Invitrogen SARL, Cergy Pontoise, la France) et du dCTP marqué au ³³P (Amersham) et ont été utilisés pour la reverse transcription des mRNA selon les recommandations des fabricants. Les échantillons d'épiderme témoins ont été retro transcrits simultanément aux échantillons strippés. Les sondes ainsi réalisées ont été purifiées sur colonne de chromatographie biospin6 (Bio-Rad, Hercule, CA, USA). L'incorporation aux sondes de ³³P a été mesurée par β-scintillation. La même quantité de sonde des échantillons d'épidermes contrôles et strippés a été utilisée pour l'hybridation sur membranes DermArray ® cDNA microarray (IntegriDerm, Birmingham, AL-, USA) selon les recommandations du fabricant.

Quantification et correction du signal: Les puces ont été analysées en utilisant un scanner Cyclone Phosphore Imager 16 bits permettant une analyse à haute résolution (Packard Instruments, Perkin-Elmer Life Sciences, Boston, MA, USA). Les images ont alors été importées dans le logiciel Imagene 5 (Biodiscovery, El Segundo, CA, USA) pour la quantification du signal puis les signaux ont été corrigés à l'aide du logiciel Genesight (Biodiscovery, El Segundo, CA, USA). Une correction locale du bruit de fond a été exécutée et les signaux positifs ont été normalisés d'une membrane contrôle par rapport à son témoin.

Analyse des données : Un ratio d'expression a été calculé divisant le signal corrigé de l'échantillon traité par le signal corrigé de l'échantillon contrôle. Un ratio moyen interindividuel a été calculé à chaque temps pour chaque gène exprimé chez au moins 50% des individus de chaque groupe.

Sélection des gènes modulés dans chaque population : On a considéré qu'un gène était modulé si son expression est significativement différente entre épidermes strippés et épidermes contrôles (test de Student, p < 0.05) ; et si au moins 50 % des volontaires du groupe présentent un ratio supérieur à 2 et aucun ne présente un ratio inférieur à 0.5 (gènes induits), et inversement pour des gènes réprimés. Enfin, ont été sélectionnés les gènes modulés au moins une fois dans le temps.

Sélection des gènes différentiellement modulés selon l'âge : La cinétique des gènes modulés à la fois chez les individus jeunes et âgés a été comparée aux temps 06h et 30h (temps auxquels les profils étaient les plus différents entre jeunes et âgés) à l'aide d'un test ANOVA à deux facteurs (facteur temps, facteur âge et interaction ; p<0.05). Les gènes dont l'expression est significativement différente selon l'âge du patient ont été sélectionnés. Une interaction significative des cinétiques reflète en plus un décalage dans le temps de la modulation des gènes.

### 2. Résultats

573 gènes sont exprimés dans l'épiderme de façon commune à tous les volontaires, qu'ils soient jeunes ou âgés. L'expression de certains d'entre eux varie consécutivement au tape stripping en comparaison à la peau témoin (expression induite ou réprimée), suivant une cinétique spécifique.

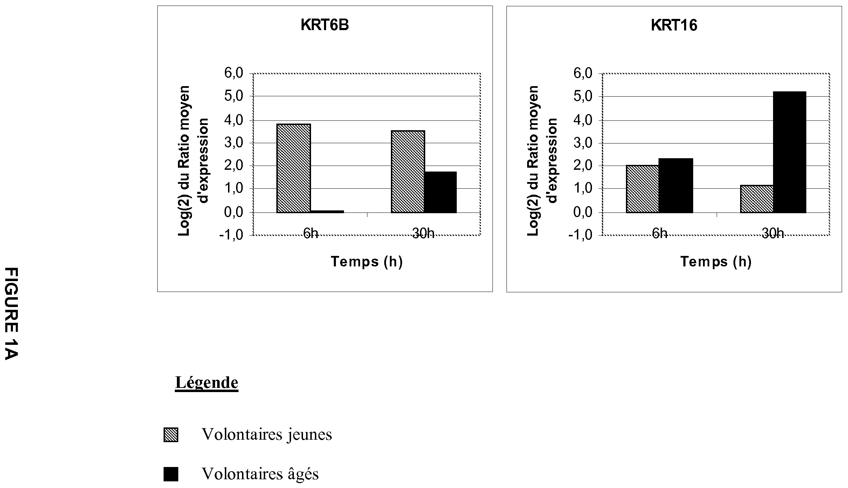

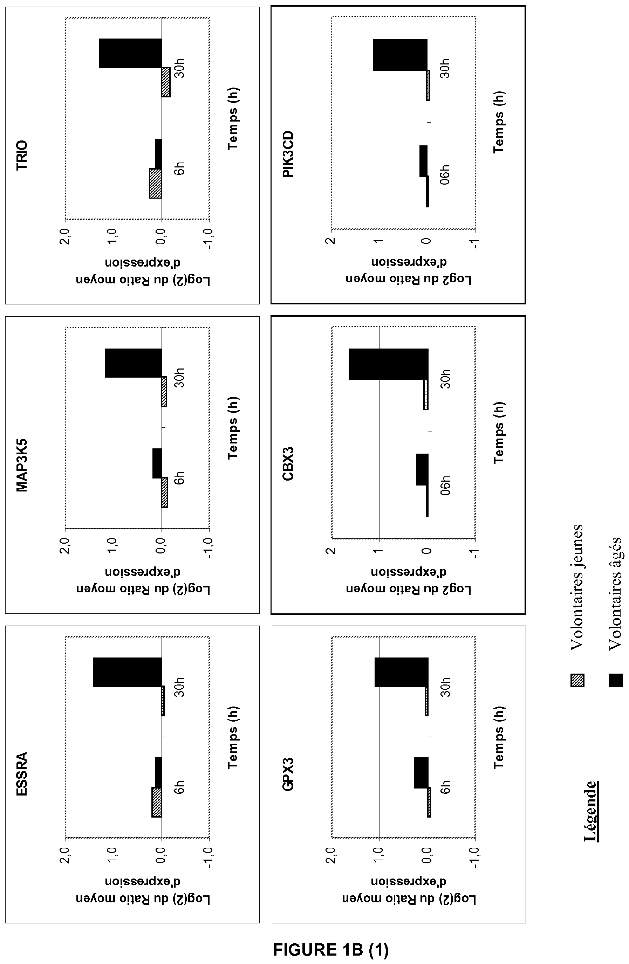

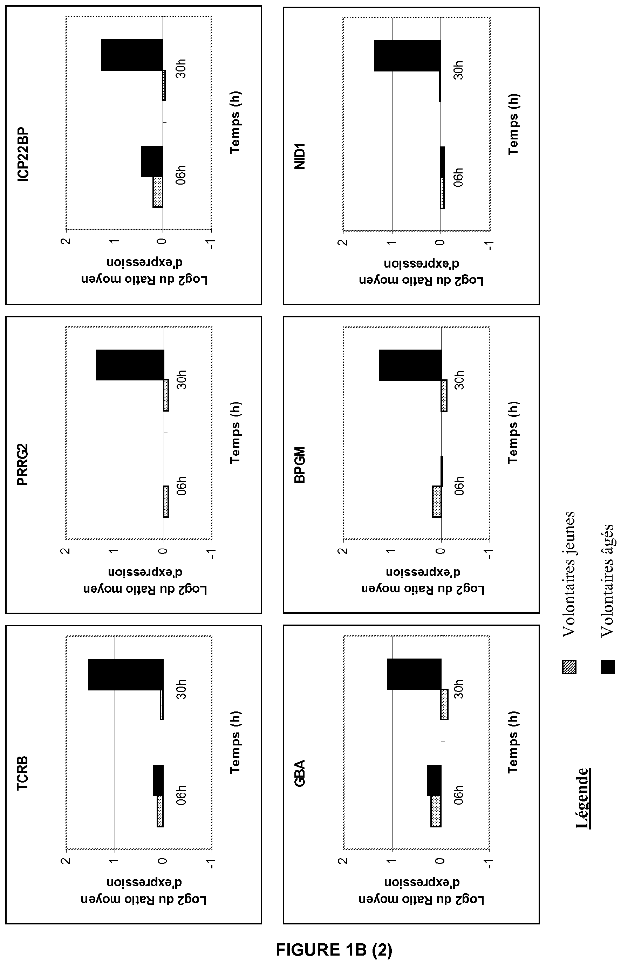

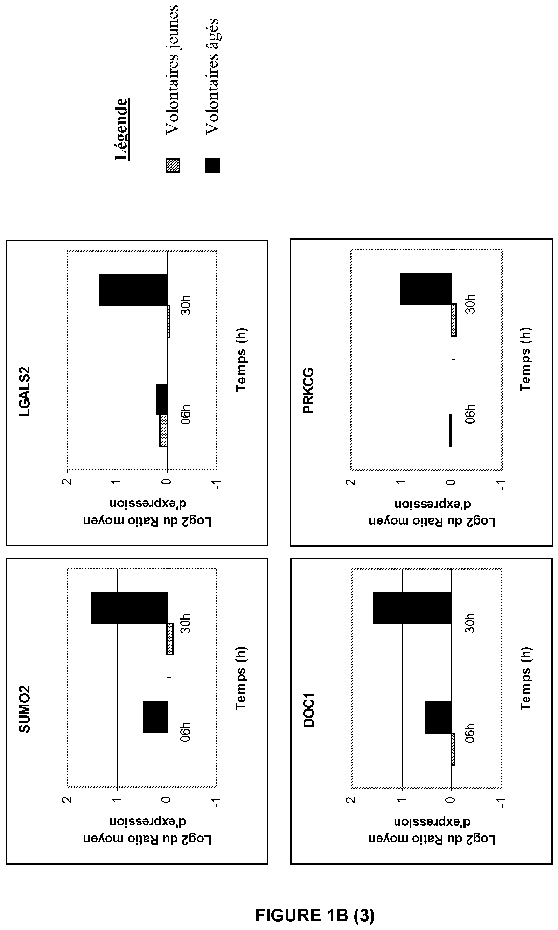

De façon surprenante et inattendue, les inventeurs ont identifié 18 gènes dont la cinétique d'expression après tape stripping est significativement différente selon l'âge du volontaire (tableau 1). En particulier, l'expression des gènes KRT6B et KRT16 est induite à 06h et à 30h dans les deux populations, mais un retard significatif dans leur cinétique de modulation a été observée chez les individus âgés (Figure 1-A). De plus, l'expression des gènes ESRRA, CBX3, MAP3K5, TRIO, PIK3CD, TCRB, PRRG2, ICP22BP, GPX3, GBA, BPGM, NID1, SMT3H2, LGALS2, DOC1, PRKCG croît de façon significative entre 06h et 30h après tape stripping chez le sujet âgé alors qu'elle reste inchangée au cours du temps chez le sujet jeune (Voir exemples en Figure 1-B).

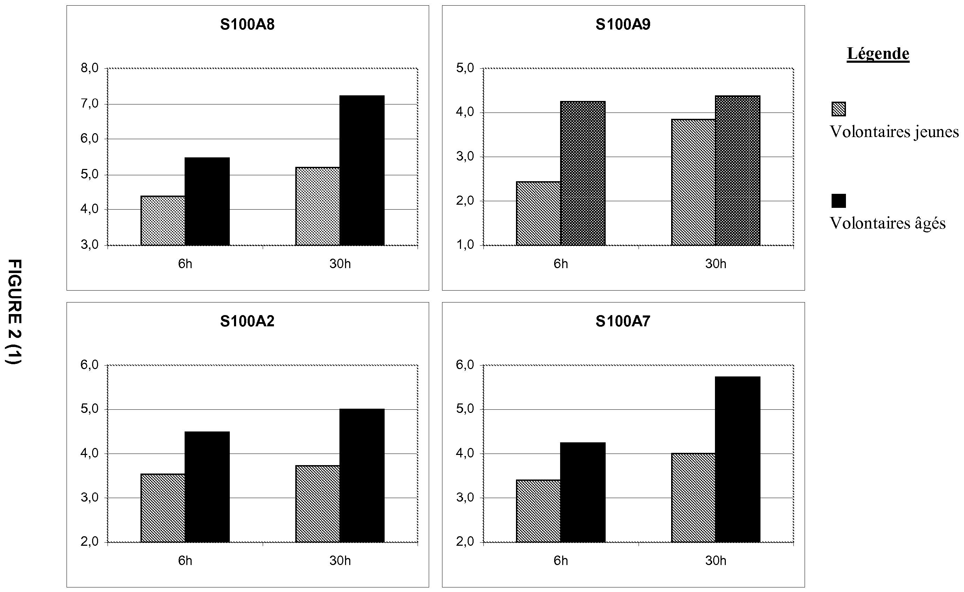

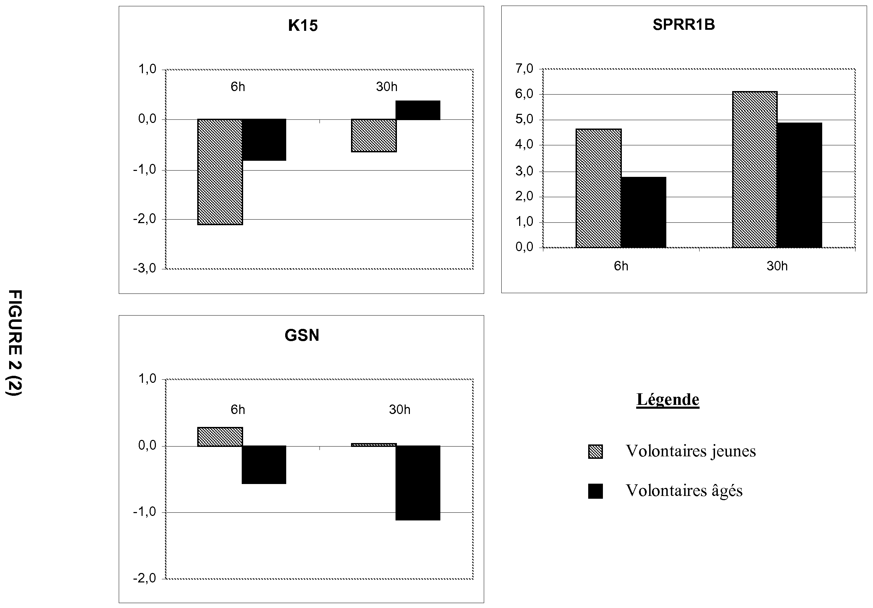

D'autre part, Il existe pour les gènes S100A8, S100A9, S100A2, S100A7, KRT15, SPRR1B et GSN (Tableau 2) une allure cinétique semblable entre 06h et 30h, mais une différence significative d'intensité de modulation allant de 1.8 log(2) à 3.12 log(2) selon que l'individu soit jeune ou âgé (Voir exemples en Figure 2).

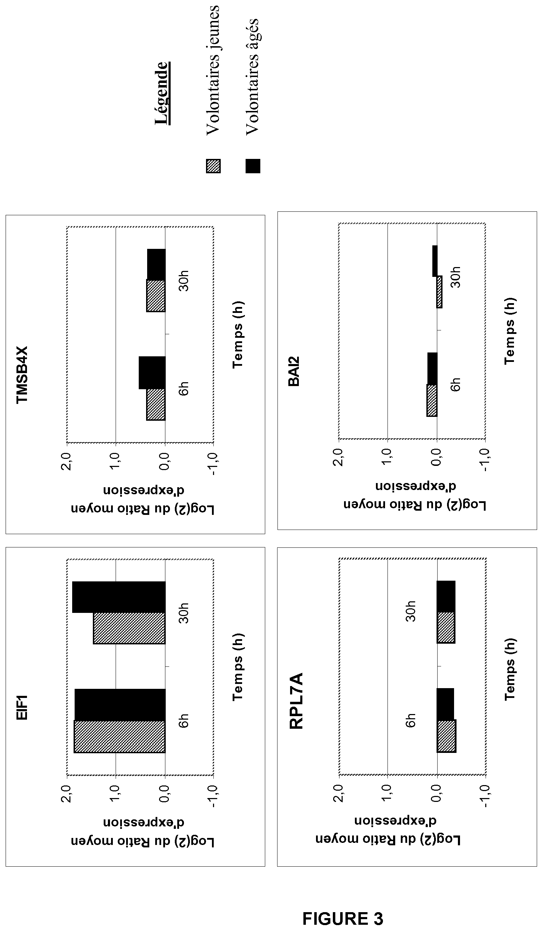

Enfin, 109 gènes n'ont montré aucune différence significative d'expression en fonction du temps ni en fonction de l'âge. Ces derniers peuvent être considérés comme gènes de ménage. Seuls 3 d'entre eux, les gènes S100A10, EIF1 et ACTR1A, ont vu leur niveau d'expression varier suite au tape stripping, cette variation étant constante dans le temps. Les autres gènes ne sont pas modulés. Les inventeurs en ont sélectionnés 19 dont le profil est proche entre jeunes et âgés. Ce sont pour la plupart des composants ribosomaux connus en tant que gènes de ménages (RPL28, RPL5, RPL35, RPS13, RPS23, RPS16, RPL18, RPS15A, RPL7A, RPS28). Les autres, G3BP1, TMSB4X, HLA-C, DYNLL1, NACA, ARHA, BAI2, UBA52, GNAS n'étaient pas connus ni utilisés en tant que gènes de ménage (Figure 3).

### 3. Conclusions

3 listes de gènes d'intérêts ont été établies :
La **Liste 1** (Tableau 1) regroupe 18 gènes dont la cinétique d'expression après tape stripping est significativement différente entre individus jeunes et âgés.
La **Liste 2** (Tableau 2) regroupe 7 gènes dont l'intensité de modulation diffère à tous les temps selon que le volontaire soit jeune ou âgé.
La **Liste 3** (Tableau 3) regroupe 22 gènes de ménage dont l'expression ne varie pas selon l'âge des volontaires.

Le niveau d'expression des gènes de la liste 1 et de la liste 2 chez les sujets âgés après tape stripping est le reflet moléculaire du disfonctionnement de la fonction homéostasique de l'épiderme dû à l'âge. L'analyse de l'expression de ces gènes constitue une solution nouvelle au problème que pose l'évaluation de l'efficacité d'actifs ou de procédés anti-âge susceptibles d'améliorer le renouvellement et l'homéostasie épidermique, et au problème que pose le diagnostic de l'état plus ou moins âgé de la peau. L'analyse de l'expression de ces gènes peut être menée et standardisée grâce à la présence de gènes de ménage listés dans le tableau 3.

**Tableau 1 : Liste des 18 gènes dont la cinétique de modulation entre 6h et 30 après tape stripping est significativement différente selon l'âge du patient :**

| **Numéro d'accession** | **Symbole du gène** | **Nom** |
|---|---|---|
| AA098896 | ESRRA | estrogen-related_receptor_alpha |
| AA132226 | CBX3 | Human_heterochromatin_protein_HP1Hs-gamma_mRNA, _complete_cds |
| AA150532 | KRT6B | keratin_6B |
| AA150828 | MAP3K5 | mitogen-activated_protein_kinase_kinase_kinase_5 |
| AA191348 | TRIO | ESTs,_Highly_similar to_(defline_not available_3522970) _[H.sapiens] |
| AA281784 | PIK3CD | phosphoinositide-3-kinase,_catalytic,_delta_polypeptide |
| AA284528 | TCRB | T-cell_receptor,_beta_cluster |
| AA430552 | PRRG2 | proline-rich_Gla_(G-carboxglutamic_acid)_polypeptide_2 |
| AA488979 | ICP22BP | cell_cycle-regulated_factor_(78_kDa) |
| AA596003 | KRT16 (ou K16) | keratin_16_(focal_non-epidermolytic_palmoplantar_keratoderma) |
| AA664180 | GPX3 | glutathione_peroxidase_3_(plasma) |
| AA670347 | GBA | glucosidase,_beta;_acid_(includes_glucosylceramidase) |
| AA678065 | BPGM | 2,3-bisphosphoglycerate_mutase |
| AA709414 | NID1 | nidogen_(enactin) |
| AA775415 | SMT3H2 | SMT3_(suppressor_of_mif_two_3,_yeast)_homolog_2 |
| AA872397 | LGALS2 | lectin,_galactoside-binding,_soluble,_2_(galectin_2) |
| R78607 | DOC1 | deleted_in_oral_cancer_(mouse,_homolog)_1 |
| R89715 | PRKCG | ESTs |

**Tableau 2 : Liste des 7 gènes dont l'intensité de modulation à 6h et 30h est significativement différente selon l'âge du patient :**

| **Numéro d'accession** | **Symbole du gène** | **Nom** |
|---|---|---|
| AA086471 | S100A8 | S100_calcium-binding_protein_A8_(calgranulin_A) |
| AA864554 | S100A9 | S100_calcium-binding_protein_A9_(calgranulin_B) |
| AA583574 | S100A7 | S100_calcium-binding_protein_A7_(psoriasin_1) |
| AA458884 | S100A2 | S100_calcium-binding_protein_A2 |
| AA878048 | KRT15 ou K15 | keratin_15 |
| AA447684 | SPRR1 B | small_proline-rich_protein_1 B_(cornifin) |
| H72028 | GSN | gelsolin_(amyloidosis,_Finnish_type) |

**Tableau 3 : Liste de 22 gènes de ménage :**

| **Numéro d'accession** | **Symbole du gène** | **Nom** |
|---|---|---|
| *AA136500* | *G3BP1* | ESTs |
| AA423800 | TMSB4X | thymosin,_beta_4,_X_chromosome |
| AA444051 | S100A10 | S100_calcium-binding_protein_A10_(annexin_ll_ligand, _calpactin_I,_light_polypeptide_(p11)) |
| AA464246 | HLA-C | major_histocompatibility_complex,_class_I,_C |
| AA464731 | EIF1 | putative_translation_initiation_factor |
| AA486746 | RPL28 | ribosomal_protein_L28 |
| AA496880 | RPL5 | ribosomal_protein_L5 |
| AA625634 | RPL35 | ribosomal_protein_L35 |
| AA629641 | RPS13 | ribosomal_protein_S13 |
| AA634008 | RPS23 | ribosomal_protein_S23 |
| AA644679 | DYNLL1 | dynein,_cytoplasmic,_light_polypeptide |
| AA664241 | NACA | -nascent-polypeptide-associated_complex_alpha_polypeptide |
| AA668301 | RPS16 | ribosomal_protein_S16 |
| AA676955 | ARHA | ras_homolog_gene_family,_member_A |
| AA775874 | RPL18 | ribosomal_protein_L18 |
| *AA777034* | *BAI2* | ESTs,_Highly_similar_to_BAI_2_[H.sapiens] |
| AA856556 | RPS28 | ribosomal_protein_S28 |
| AA872341 | RPS15A | ribosomal_protein_S15a |
| AA878561 | U BA52 | proteasome_(prosome,_macropain)_subunit,_alpha_type,_7 |
| H23422 | RPL7A | ribosomal_protein_L7a |
| R40850 | ACTR1A | H.sapiens_mRNA_for_alpha-centractin |
| *R43581* | *GNAS* | Human_guanine_nucleotide-binding_protein_G-s,_alpha_subunit _mRNA,_partial_cds |

### REFERENCES

1. Barel, A. O. and Clarys, P.; Study of the stratum corneum barrier function by transepidermal water loss measurements: comparison between two commercial instruments: Evaporimeter and Tewameter; 1995; Skin Pharmacol; 8 (186-195)
2. Denda, M.; New stratégies to improve skin barrier homeostasis; 1-11-2002; Adv Drug Deliv Rev; 54 Suppl 1 (S123-S130)
3. Ghadially, R., et al.; The aged epidermal permeability barrier. Structural, functional, and lipid biochemical abnormalities in humans and a senescent murine model; 1995; J Clin Invest; 95 (2281-2290)
4. Grubauer, G., Feingold, K. R., and Elias, P. M.; Relationship of epidermal lipogenesis to cutaneous barrier function; 1987; J Lipid Res; 28 (746-752)
5. Grubauer, G., et al.; Lipid content and lipid type as déterminants of the epidermal permeability barrier; 1989; J Lipid Res; 30 (89-96)
6. Kuss, O. and Diepgen, T. L.; Proper statistical analysis of transepidermal water loss (TEWL) measurements in bioengineering studies; 1998; Contact Dermatitis; 39 (64-67)
7. Leveque, J. L.; Quantitative assessment of skin aging; 2001; Clin Geriatr Med; 17 (673-89, vi)
8. Lock-Andersen, J., et al.; Epidermal thickness, skin pigmentation and constitutive photosensitivity; 1997; Photodermatol Photoimmunol Photomed; 13 (153-158)
9. Marionnet, C., et al.; Modulation of gene expression induced in human epidermis by environmental stress in vivo; 2003; J Invest Dermatol; 121 (1447-1458)
10. Menon, G. K., et al.; De novo sterologenesis in the skin. II. Regulation by cutaneous barrier requirements; 1985; J Lipid Res; 26 (418-427)
11. Piaserico, S.,et al.; Allergic contact sensitivity in elderly patients; 2004; Aging Clin Exp Res; 16 (221-225**)**
12. Piepkorn, M., Lo, C., and Plowman, G.; Amphiregulin-dependent proliferation of cultured human keratinocytes: autocrine growth, the effects of exogenous recombinant cytokine, and apparent requirement for heparin-like glycosaminoglycans; 1994; J Cell Physiol; 159 (114-120)
13. Pinnagoda, J., Tupker, R. A., Agner, T., and Serup, J.; Guidelines for transepidermal water loss (TEWL) measurement. A report from the Standardization Group of the European Society of Contact Dermatitis; 1990; Contact Dermatitis; 22 (164-178)
14. Proksch, E., et al.; Barrier function regulates epidermal DNA synthesis; 1991; J Clin Invest; 87 (1668-1673)
18. Tanaka, M., Zhen, Y. X., and Tagami, H.; Normal recovery of the stratum corneum barrier function following damage induced by tape stripping in patients with atopic dermatitis; 1997; Br J Dermatol; 136 (966-967)
19. Treffel, P., et al.; Hydration, transepidermal water loss, pH and skin surface parameters: correlations and variations between dominant and non-dominant forearms; 1994; Br J Dermatol; 130 (325-328)
20. Van, Sam, V, et al.; TEWL measurement standardization: kinetic and topographic aspects; 1994; Acta Derm Venereol; 74 (168-170)
21. Wood, L. C., et al.; Cutaneous barrier perturbation stimulates cytokine production in the epidermis of mice; 1992; J Clin Invest; 90 (482-487)
22. Zhai, H., Leow, Y. H., and Maibach, H. I.; Human barrier recovery after acute acetone perturbation: an irritant dermatitis model; 1998; Clin Exp Dermatol; 23 (11-13)

## Revendications

1. Méthode d'évaluation in vitro de l'homéostasie épidermique de la peau d'un sujet humain comprenant l'analyse différentielle de l'expression des gènes ESRRA, CBX3, TRIO, TCRB, PRRG2, ICP22BP, GPX3, GBA, BPGM, SMT3H2, LGALS2, DOC1 et GSN en réponse à une agression physique ou chimique du stratum corneum.

2. Méthode d'évaluation de l'homéostasie épidermique de la peau d'un sujet humain selon la revendication 1, comprenant la comparaison :
- de la cinétique de modulation de l'expression des gènes ESRRA, CBX3, TRIO, TCRB, PRRG2, ICP22BP, GPX3, GBA, BPGM, SMT3H2, LGALS2 et DOC1 au sein de l'épiderme correspondant à une région de la peau après une agression physique ou chimique du stratum corneum et
- du niveau de modulation de l'expression du gène GSN au sein de l'épiderme correspondant à une région de la peau après une agression physique ou chimique du stratum corneum,
à la cinétique de référence ou au niveau de référence pour la modulation de l'expression desdits gènes.

3. Méthode selon la revendication 2 où la modulation de l'expression des gènes est déterminée par référence à une autre région de la peau n'ayant pas subi l'agression.

4. Méthode selon la revendication 2 où la modulation de l'expression des gènes est normalisée par référence à l'expression d'au moins un gène choisi parmi S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 et GNAS au sein de l'épiderme correspondant à la région agressée.

5. Méthode selon la revendication 1 ou 2, où ladite agression physique ou chimique du stratum corneum est un tape stripping, l'application d'acétone, d'un agent délipidant, d'un agent chimique abrasif, ou bien une méthode physique telle que la chaleur.

6. Méthode selon la revendication 2 où la cinétique de référence ou le niveau de référence correspond à la cinétique ou au niveau de modulation de l'expression desdits gènes observé normalement pour une peau d'un sujet âgé d'environ 25 ans.

7. Méthode selon la revendication 1 ou 2, comprenant l'analyse différentielle de l'expression des gènes KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN.

8. Méthode selon la revendication 1 ou 2 où ladite analyse différentielle ou ladite comparaison est réalisée dans les 72 heures suivant l'agression physique ou chimique, de préférence dans les 36 heures.

9. Méthode de diagnostic de l'état de l'épiderme d'un individu humain comprenant l'évaluation de l'homéostasie épidermique de la peau dudit individu selon l'une des revendications 1 à 8.

10. Méthode selon la revendication 9, où la peau diagnostiquée est qualifiée de « peau âgée » si l'analyse différentielle de l'expression d'au moins l'un des gènes choisi parmi ESRRA, CBX3, TRIO, TCRB, PRRG2, ICP22BP, GPX3, GBA, BPGM, SMT3H2, LGALS2 et DOC1, s'écarte de plus de 50% de la modulation moyenne normalement observée pour une peau d'un sujet âgé d'environ 25 ans, de préférence d'au moins deux ou trois gènes, de préférence d'au moins cinq gènes, de préférence d'au moins 10 gènes.

11. Procédé de détermination de l'efficacité d'un traitement de l'épiderme d'un individu humain, comprenant l'évaluation de l'homéostasie épidermique de la peau avant et après traitement par une méthode selon l'une des revendications 1 à 8.

12. Procédé de criblage de molécules pour leur action bénéfique sur l'homéostasie épidermique, comprenant l'évaluation de l'homéostasie épidermique de la peau d'un individu humain par une méthode selon l'une des revendications 1 à 8, avant et après application de la molécule à cribler.

13. Procédé pour tester des traitements pour leur effet bénéfique sur la barrière homéostasique de l'épiderme humain, comprenant l'évaluation de la fonction homéostasique de la peau d'un individu humain par une méthode selon l'une des revendications 1 à 8, avant et après mise en oeuvre du traitement à tester.

14. Procédé selon l'une des revendications 11 à 13, où le traitement ou la molécule testée est considéré comme efficace si il est observé :
- une inhibition de l'induction après 30 heures d'au moins un gène choisi parmi ESRRA, CBX3, TRIO, TCRB, PRRG2, ICP22BP, GPX3, GBA, BPGM, SMT3H2, LGALS2 et DOC1; et/ou
- une modification de l'intensité de modulation du gène GSN, ladite modification réduisant la différence entre l'intensité de modulation observée et l'intensité de modulation de référence caractéristique de la peau d'un sujet âgé d'environ 25 ans.

## Patentansprüche

1. In-vitro-Verfahren zur Beurteilung der epidermalen Homöostase der Haut einer menschlichen Person, umfassend die Differentialanalyse der Expression der Gene ESRRA, CBX3, TRIO, TCRB, PRRG2, ICP22BP, GPX3, GBA, BPGM, SMT3H2, LGALS2, DOC1 und GSN als Reaktion auf eine physische oder chemische Einwirkung auf das Stratum corneum.

2. Verfahren zur Beurteilung der epidermalen Homöostase der Haut eines Menschen nach Anspruch 1, umfassend den Vergleich
- der Modulationskinetik der Expression der Gene TCRB, PRRG2, ICP22BP, GPX3, GBA, BPGM, SMT3H2, LGALS2 und DOC1 in der Epidermis, die einer Hautregion nach einer physischen oder chemischen Einwirkung auf das Stratum corneum entspricht, und
- des Modulationsniveaus der Expression des Gens GSN in der Epidermis, die einer Hautregion nach einer physischen oder chemischen Einwirkung auf das Stratum corneum entspricht,
mit der Referenzkinetik oder dem Referenzniveau hinsichtlich der Expressionsmodulation dieser Gene.

3. Verfahren nach Anspruch 2,
bei dem die Expressionsmodulation der Gene in Bezug auf eine andere Hautregion bestimmt wird, die keiner Einwirkung ausgesetzt war.

4. Verfahren nach Anspruch 2,
bei dem die Expressionsmodulation der Gene vereinheitlicht ist in Bezug auf die Expression von wenigstens einem Gen ausgewählt aus S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 und GNAS in der Epidermis, die der Region entspricht, die der Einwirkung ausgesetzt ist.

5. Verfahren nach Anspruch 1 oder 2,
bei dem die physische oder chemische Einwirkung auf das Stratum corneum ein Tape Stripping, die Applikation von Aceton, eines entfettenden Wirkstoffs, eines abrasiven chemischen Wirkstoffs oder ein physisches Verfahren wie zum Beispiel Wärme ist.

6. Verfahren nach Anspruch 2,
bei dem die Referenzkinetik oder das Referenzniveau der Kinetik oder dem Niveau der Expressionsmodulation dieser Gene entspricht, das normalerweise bei einer Haut einer Person im Alter von etwa 25 festgestellt wird.

7. Verfahren nach Anspruch 1 oder 2,
umfassend die Differentialanalyse der Expression der Gene KRT6B, KRT16, ESRRA,CBX3, MAP3K5, TRIO, PIK3CD, TCRB, PRRG2, ICP22PB, GPX3, GBA, BPGM, NID1, SMT3H2, LGALS2, DOC1, PRKCG, S100A8, S100A9, S100A2, S100A7, K15, SPRR1B und GSN.

8. Verfahren nach Anspruch 1 oder 2,
bei dem die Differentialanalyse oder der Vergleich innerhalb von 72 Stunden nach der erfolgten physischen oder chemischen Einwirkung, vorzugsweise innerhalb von 36 Stunden, durchgeführt wird.

9. Verfahren zur Diagnose des Zustands der Epidermis einer menschlichen Person, umfassend die Beurteilung der epidermalen Homöostase der Haut dieser Person nach einem der Ansprüche 1 bis 8.

10. Verfahren nach Anspruch 9,
bei dem die diagnostizierte Haut als "gealterte Haut" bezeichnet wird, wenn die Differentialanalyse der Expression von wenigstens einem der Gene ausgewählt aus ESRRA, CBX3, TRIO, TCRB, PRRG2, ICP22BP, GPX3, GBA, BPGM, SMT3H2, LGALS2 und DOC1 um mehr als 50 % von der durchschnittlichen Modulation abweicht, die normalerweise für eine Haut einer Person im Alter von etwa 25 Jahren festgestellt wird, vorzugsweisen von wenigstens zwei oder drei Genen, vorzugsweise von wenigstens fünf Genen, vorzugsweise von wenigstens zehn Genen.

11. Verfahren zur Bestimmung der Wirksamkeit einer Behandlung der Epidermis einer menschlichen Person, umfassend die Beurteilung der epidermalen Homöostase der Haut vor und nach der Behandlung durch ein Verfahren nach einem der Ansprüche 1 bis 8.

12. Verfahren zum Molekül-Screening hinsichtlich deren Wirkung auf die epidermale Homöostase, umfassend die Beurteilung der epidermalen Homöostase der Haut einer menschlichen Person durch ein Verfahren nach einem der Ansprüche 1 bis 8 vor und nach Applikation des zu screenenden Moleküls.

13. Verfahren zum Testen von Behandlungen hinsichtlich ihrer positiven Auswirkung auf die homöostatische Barriere der menschlichen Epidermis, umfassend die Beurteilung der homöostatischen Funktion der Haut einer menschlichen Person durch ein Verfahren nach einem der Ansprüche 1 bis 8 vor und nach Durchführung der zu testenden Behandlung.

14. Verfahren nach einem der Ansprüche 11 bis 13, bei dem die Behandlung oder das zu testende Molekül als wirksam angesehen wird,
- wenn nach 30 Stunden eine Induktionshemmung von wenigstens einem Gen ausgewählt aus ESRRA, CBX3, TRIO, TCRB, PRRG2, ICP22BP, GPX3, GBA, BPGM, SMT3H2, LGALS2 und DOC1 festgestellt wird; und/oder
- wenn eine Veränderung der Modulationsintensität des Gens GSN festgestellt wird, wobei die Veränderung den Unterschied zwischen der festgestellten Modulationsintensität und der Referenzmodulationsintensität verringert, die für die Haut einer Person im Alter von etwa 25 Jahren charakteristisch ist.

## Claims

1. A method for in vitro evaluating the epidermal homeostasis of the skin of a subject, comprising differential analysis of the expression of the genes ESRRA, CBX3, TRIO, TCRB, PRRG2, ICP22BP, GPX3, GBA, BPGM, SMT3H2, LGALS2, DOC1 and GSN in response to a physical or chemical challenge of the stratum corneum.

2. A method for evaluating the epidermal homeostasis of the skin of a subject according to claim 1, comprising comparing:
• the kinetics of modulation of the expression of the genes ESRRA, CBX3, TRIO, TCRB, PRRG2, ICP22BP, GPX3, GBA, BPGM, SMT3H2, LGALS2 and DOC1 in the epidermis corresponding to a region of the skin following a physical or chemical challenge to the stratum corneum; or
• the level of modulation of the expression of the gene GSN in the epidermis corresponding to a region of the skin after a physical or chemical challenge of the stratum corneum;
with the reference kinetics or reference level for modulation of the expression of said genes.

3. A method according to claim 2, wherein the modulation of the expression of the genes is determined with reference to another region of the skin which has not been subjected to the challenge.

4. A method according to claim 2, wherein the modulation of the expression of the genes is normalized with respect to the expression of at least one gene selected from the group consisting of S100A10, EIF1, ACTR1A, RPL28, RPL5, RPL35, RPS13, RPS23, RPS16, RPL18, RPS15A, RPL7A, RPS28, G3BP1, TMSB4X, HLA-C, DYNLL1, NACA, ARHA, BAI2, UBA52 and GNAS in the epidermis corresponding to the challenged region.

5. A method according to claim 1 or claim 2, wherein said physical or chemical challenge of the stratum corneum is tape stripping, the application of acetone, a delipidation agent, an abrasive chemical agent or a physical method such as heat.

6. A method according to claim 2, wherein the reference kinetics or the reference level corresponds to the kinetics or the level of modulation of the expression of said genes normally observed for skin of a subject of approximately 25 years of age.

7. A method according to claim 1 or 2, comprising the differential analysis of the expression of the genes KRT6B, KRT16, ESRRA, CBX3, MAP3K5, TRIO, PIK3CD, TCRB, PRRG2, ICP22BP, GPX3, GBA, BPGM, NID1, SMT3H2, LGALS2, DOC1, PRKCG, S100A8, S100A9, S100A2, S100A7, K15, SPRR1B et GSN.

8. The method according to claim 1 or claim 2, wherein said differential analysis or said comparison is carried out within 72 hours following the physical or chemical challenge, preferably within 36 hours.

9. A method for diagnosing the epidermis condition of a human individual, comprising evaluating the epidermal homeostasis of the skin of said individual according to any one of claims 1 to 8.

10. The method according to claim 9, wherein the diagnosed skin is qualified as "aged skin" if the differential analysis of the expression of at least one of the genes selected from the group consisting of ESRRA, CBX3, TRIO, TCRB, PRRG2, ICP22BP, GPX3, GBA, BPGM, SMT3H2, LGALS2 and DOC1 differs by more than 50% from the mean modulation normally observed for skin of a subject approximately 25 years of age, preferably at least two or three genes, preferably at least five genes, more preferably at least 10 genes.

11. A method for determining the effectiveness of a treatment of the epidermis of a human individual, comprising evaluating the epidermal homeostasis of the skin before and after treatment using a method according to any one of claims 1 to 8.

12. A method for screening molecules for their beneficial action on epidermal homeostasis, comprising evaluating the epidermal homeostasis of the skin of a human individual using a method according to any one of claims 1 to 8, before and after application of the molecule to be screened.

13. A method for testing treatments for their beneficial effect on the homeostatic barrier of the human epidermis, comprising evaluating the homeostatic function of the skin of a human individual using a method according to any one of claims 1 to 8, before and after carrying out the treatment to be tested.

14. A method according to any one of claims 11-13, wherein the treatment or tested molecule is considered to be effective if the following is observed:
• an inhibition of induction after 30 hours of at least one gene selected from the group consisting of ESRRA, CBX3, TRIO, TCRB, PRRG2, ICP22BP, GPX3, GBA, BPGM, SMT3H2, LGALS2 and DOC1; and/or
• a modification of the intensity of modulation of the gene GSN, said modification reducing the difference between the observed intensity of modulation and the reference modulation intensity characteristic of the skin of a subject of approximately 25 years of age.
